# EUROPEAN PATENT SPECIFICATION

(11) **EP 0 875 225 B1**
(45) Date of publication and mention of the grant of the patent: **05.02.2003**
(21) Application number: 97107169.1
(22) Date of filing: 30.04.1997
(51) Int. Cl.: A61F 13/15

(54) **Individual layered structures containing particulate material, and process for manifacturing said structures**
Pulverförmiges Material enthaltende individuelle Schichtstrukturen, und deren Herstellungsverfahren
Structures individuelles en couches contenant un matériau sous forme de poudre et leur procédé de fabrication

(43) Date of publication of application: 04.11.1998
(73) Proprietor: THE PROCTER & GAMBLE COMPANY, Cincinnati, Ohio 45202 (US)
(72) Inventor: Febo, Pietro, 65126 Pescara (IT); Gagliardi, Ivano, 65123 Pescara (IT); Wessel, Thomas, 65100 Pescara (IT)
(74) Representative: Kremer, Véronique

(56) References cited:
- EP-A- 0 022 792
- WO-A-95/17868
- DE-A- 3 002 136
- US-A- 3 709 221
- US-A- 3 748 207
- US-A- 4 715 918

## Description

### FIELD OF THE INVENTION

The present invention relates to individual layered structures for use in a disposable absorbent article and comprising a particulate material, wherein the loss or spillage of the particulate material from the structures is prevented, and to a process for manufacturing such layered structures. Preferably, the individual layered structures comprise absorbent fibrous layers and can be used as absorbent elements in disposable absorbent articles such as absorbent articles for incontinent adults, babies' nappies, sanitary towels, dressings and the like.

### BACKGROUND OF THE INVENTION

Disposable absorbent articles are well known and all have absorbent elements for absorbing and retaining body fluids; an absorbent element must be able to acquire liquid rapidly and to distribute it internally so as to prevent leakage and must also have a good capacity to retain the fluids when subjected to the normal pressures of use.

Absorbent elements made mainly of hydrophilic fibrous material such as, for example, pads of cellulose fibres, layers of wadding, or the like generally have satisfactory characteristics as regards their liquid-absorption rate and can distribute the liquid effectively within them but are very ineffective from the point of view of retention when subjected to the normal pressures of use.

The use of absorbent gelling materials in combination with hydrophilic fibres in order to increase the absorption and retention capacities of the absorbent elements is known.

Absorbent gelling materials, commonly known as superabsorbents, are polymers which can swell up and absorb large quantities of liquid, particularly water, or also, to a lesser extent, body fluids.

They also have the particular property that they retain the fluids even under moderate pressure; owing to this characteristic, their use in absorbent elements for disposable absorbent articles has been proposed for some time.

With the use of absorbent gelling materials, it is possible to produce absorbent elements which contain less hydrophilic fibres for a given absorption capacity and which consequently have smaller dimensions, particularly thicknesses, than conventional absorbent elements made of fibres alone.

Absorbent gelling materials are commonly incorporated in particle form within fibrous structures. Structures have been formed in which the fibres and the particles of hydrogelling, absorbent material are disposed in separate, generally very thin, superposed layers or, alternatively, the particles can also be mixed with fibres in one of the fibrous layers.

Many particular forms of layered, absorbent structures of this type, in which the fibrous material is represented by one of more layers of wadding, absorbent paper or non-woven fabric, and in which the particles of absorbent gelling material are incorporated in the structure in various ways, are known in the art.

Different types of particulate materials, other than absorbent gelling material particles, can also be incorporated in layered absorbent structures, such as for example odour control materials in particle or powder form.

Usually a continuous, web-like layered absorbent structure is manufactured, which is subsequently cut in smaller pieces to provide the individual layered absorbent structures to be used as absorbent elements in disposable absorbent articles.

Layered absorbent structures incorporating particulate material can be formed directly on the production line for the absorbent articles in which they are to be incorporated, or, alternatively, they can be produced independently as semi-finished products that are sold and stored separately in form of a continuous web-like structure, e.g. wound in a roll, or otherwise held in a container, and are then fed to the production line.

A common problem with layered absorbent structures incorporating particulate material consists in the effective containment of the particles within the structure in a stable manner, e.g. avoiding that the particles move within the structure and become locally concentrated. The particulate material can also escape from the edges of the layered structure, particularly along the edges where the individual structures are cut from the continuous web-like structure, therefore creating a problem both in the production line and in the final product.

A known solution for the formation of a layered absorbent structure provides for the use of an adhesive, e.g. of the hot melting type, applied to the entire surface of one of the fibrous layers with the dual purpose of bonding the two fibrous layers together and simultaneously fixing the particles of e.g. absorbent gelling material and/or odour control material between them.

The use of an adhesive may, however, affect the characteristics both of the fibrous layer to which the adhesive is applied, and of the particulate material which come into contact with the adhesive.

In general, therefore, it is necessary not to use an excessive quantity of adhesive and consequently the possibility of the loss of particulate material from the edges of the layered structure cannot be completely eliminated.

Different solutions to the problem of the loss of particulate material along the longitudinal edges of a continuous, web-like layered absorbent structure are known, by longitudinal edges being meant the edges of the layered structure that are parallel to the direction of formation of the structure itself.

For example, the continuous, web-like, layered absorbent structure can be completely surrounded with a layer of wadding, or, alternatively, the structure can be provided by means of a single layer of fibrous material on which the adhesive and the particulate material are distributed only on a central longitudinal strip and subsequently the two side portions are folded so that they partially overlap approximately along the longitudinal axis, and are joined e.g. by means of adhesive.

In International Patent Applications WO 94/01069 and WO 95/17868 by Procter & Gamble Company continuous, web-like, thin, layered absorbent structures containing absorbent gelling material for use as absorbent elements in disposable absorbent articles are described. Such layered absorbent structures are formed by at least two fibrous layers comprising between them a layer of particles of absorbent gelling material, the two fibrous layers being joined together by the melting of particles of thermoplastic, polymeric, organic material in finely divided solid form distributed and mixed with the absorbent gelling material, and by two lines of adhesive disposed along the longitudinal edges of the structure.

While the above described layered structures do not have the problem of loss of particulate material along the longitudinal edges, they still have a problem of loss or spillage of particles when they are cut transversely relative to the longitudinal direction in the production line in order to form the individual layered absorbent structures that are to be incorporated as absorbent elements in the absorbent articles. Not only in fact the transverse cuts open the layered structure, therefore exposing the particulate material comprised therebetween, but the cutting action itself tends to break the particles with formation of powders and, which is more likely to occur in the layered absorbent structures described in the two above mentioned international applications, it can also break the bonding points created by the melted polymeric material between the particles and the fibrous layers. The problem of the loss of particulate material along the cut edges is therefore enhanced by these effects.

U.S. Patent 3,748,207 describes a continuous structure from which individual diapers can be formed by cutting them therefrom. The structure has topsheet and backsheet layers enclosing individual absorbent cores. Topsheet and backsheet are in tum heat sealed in correspondence of the cut zone by the addition of a heat sealable film strip intermediate the topsheet and the backsheet.

U.S. Patents 4,715,918 by Kimberly-Clark Corporation and 4,646,510 by Acumeter Lab. Inc., and European Patent EP-B-22792 by Beghin Say SA describe layered structures with particles embedded within two fibrous layers and confined in so called pockets or pouches which are created by the two fibrous layers joined by means e.g. of adhesive or fibre entanglement. The particles are to be selectively deposited on the substrate only on predetermined zones in order to form the pockets or pouches. These methods are capable of achieving a structure sealed both in longitudinal and in transverse direction, but are rather complex and are not suitable to produce pre-formed laminated structures that are to be subsequently fed to a production line and cut at predetermined intervals to form the individual layered absorbent structures.

It is therefore an object of the present invention to provide individual layered structures that comprise a particulate material between containing layers that do not have the problem of loss or spillage of the particulate material from the edges thereof.

It is a further object of the present invention to provide a process for manufacturing individual layered structures comprising a particulate material, which process prevents the loss or spillage of particulate material from the structures incorporating such material, particularly during the cutting step by which the individual layered structures are cut from a larger layered structure, e.g. a continuous, web-like layered structure.

It is still a further object of the present invention to provide such a process that is applicable both to layered structures that are formed directly on the production line for the articles in which they are to be incorporated, and to continuous, web-like, layered structures that are produced as semi-finished intermediate products and are intended to be later fed to the production line.

### SUMMARY OF THE INVENTION

The present invention relates to individual layered structures for use in a disposable absorbent article and comprising a particulate material contained between containing layers, at least one of the layers being permeable to liquids. The individual layered structure is cut from a larger structure and is joined along at least part of the cut, and further has end surfaces along said cuts. The individual layered structure comprises at least one patch of material applied along at least part of the end surfaces, wherein the at least one patch of material providing the containing layers with joining means in the at least part of the end surfaces where it is applied.

The invention further relates to a process for manufacturing the individual layered structures by cutting them from a larger structure, and then providing them with at least one patch of material applied along at least part of the end surfaces formed along the cut, the at least one patch of material being capable of providing the containing layers with joining means in the region of at least part of the end surfaces where it is applied.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a perspective view of a continuous, web-like, layered structure that can be employed to form individual layered structure according to the present invention;
FIG. 2 is a perspective view of a continuous, web-like, layered structure of the type illustrated in FIG. 1, wound in a roll;
FIG. 3 is a perspective view of an individual layered structure being made from a continuous, web-like layered structure in accordance with the present invention;
FIGS. 4a, 4b, 4c, and 4d are cross-sectional views of a portion of the individual layered structure shown in FIG. 3 comprising an end surface, as taken along a section line corresponding to the longitudinal centreline A-A, showing four different alternative embodiments for the applied patch of material; embodiments illustrated in FIGS. 4c and 4d also comprise a further layer adjacent to the layered structure;
FIG. 5 is a schematic flow diagram of a process for making individual layered absorbent structures and for incorporating them in absorbent articles according to the present invention;
FIG. 6 is a schematic flow diagram of an alternative process for making individual layered absorbent structures and for incorporating them in absorbent articles according to the present invention.

### DETAILED DESCRIPTION OF THE INVENTION

The invention relates to individual layered structures for use in a disposable absorbent article that are cut from a larger structure and that comprise a particulate material within containing layers, at least one of which layers is liquid permeable, that are joined along the cut so as to prevent loss or spillage of the particulate material from the edges thereof. The invention further relates to a method for manufacturing said structures from a continuous, web-like, layered structure. In a preferred embodiment, the individual layered structures comprise fibrous layers that are preferably liquid absorbent. The individual layered structures of the present invention will be described herein in relation to their use as absorbent elements in disposable absorbent articles, by "disposable absorbent articles", as used herein, being actually meant for conciseness "disposable sanitary absorbent articles", as explained in more detail below, but they can be used for different purposes, for example as an absorbent structure for different types of disposable absorbent articles, e.g. for a disposable cleaning article.

Disposable sanitary absorbent articles, or, more briefly, disposable absorbent articles, are intended to be articles that are worn by the user in direct contact with the body; their purpose is to absorb body fluids and they are then thrown away after a single use.

The individual layered structures of the present invention can constitute integrally the absorbent element of a disposable absorbent article, or they can be comprised therein as part of the absorbent element, or in any case they can constitute an element of a disposable absorbent article.

Disposable absorbent articles, such as for example sanitary napkins, pantiliners, incontinent pads, or diapers, typically comprise a fluid pervious topsheet, a fluid impervious backsheet, that can optionally be water vapour and/or gas pervious, and an absorbent element comprised therebetween.

The term "layered", as used herein, indicates any structure in which layers containing particulate material are recognizable from layers that substantially do not comprise particulate material. This comprises structures in which the containing layers and the particulate material are disposed in separate, superposed layers or, alternatively, structures in which the particulate material is embedded in a given position within the thickness of a single layer, e.g. a fibrous layer, for example in correspondence of the centre of the layer, in order to prevent particulate material from escaping from one or both major flat surfaces of the layered structure.

The term "cutting line", as used herein, indicates a path where the continuous web-like layered structure has to be cut in order to provide individual layered structures. A cutting line can comprise a closed perimeter or, alternatively or in combination, cutting lines that are separate from each other.

The structure can also comprise non fibrous containing layers, for example a polymeric film layer, provided that at least one of the containing layers is liquid permeable.

The individual layered absorbent structures of the present invention and the process for manufacturing said individual layered absorbent structures, will be herein described with reference to a continuous, web-like, layered absorbent structure which is similar to the thin layered absorbent structures described in the two above cited international applications WO 94/1069 and WO 95/17868.

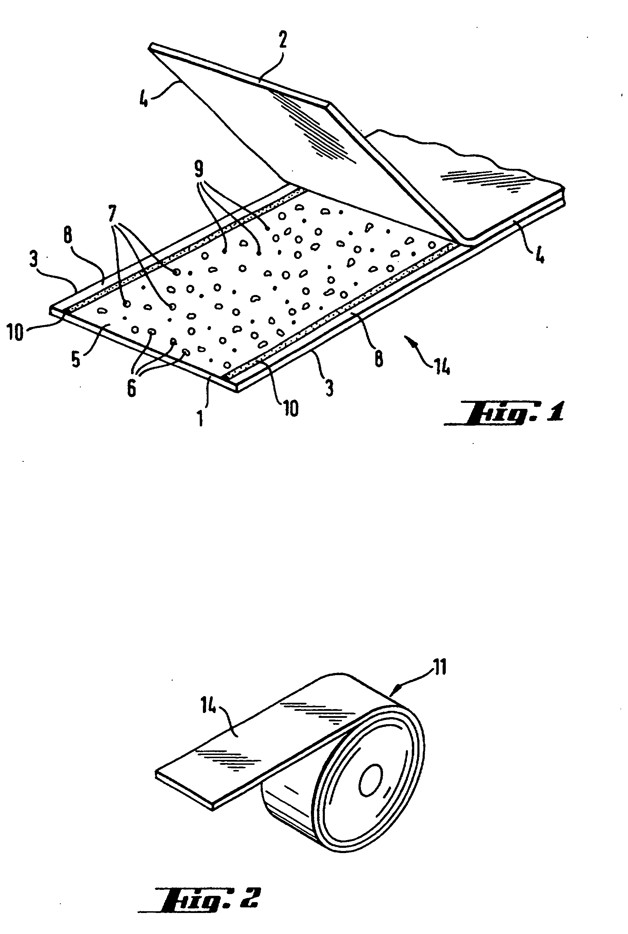

Figure 1 shows a continuous, web-like, layered absorbent structure 14 with one of the layers partially raised to show its construction more clearly.

In Figure 1 it is possible to distinguish a first fibrous layer 1 and a second fibrous layer 2 in the form of two continuous strips of the same width, which are superposed so that their respective longitudinal edges 3 and 4 coincide; the fibrous layers constitute the containing layers and can be made of various materials such as, for example, paper, wadding, or non-woven fabric; they are preferably made of dry-formed layers, generally referred to as "air laid" layers, of short cellulose fibres having a basic weight of between 20 g/m² and 150 g/m².

Alternatively, the two fibrous layers can be made of different materials, for example the second fibrous layer 2 may consist of a dry-formed mixture of cellulose fibres and bicomponent polyethylene/polypropylene fibres, such as, for example, those sold by Danaklon a/s of Varde, Denmark, as AL-Thermal B and AL-Thermal C.

Between the two containing fibrous layers 1 and 2 there is an intermediate layer 5 of particulate material made of a mixture of particles of hydrogelling, absorbent material 6, particles of odour control material 9 and a thermoplastic, polymeric, organic material in finely divided solid form, preferably in form of particles 7; the width of the said intermediate layer 5 is less than that of the outer two fibrous layers 1 and 2 which extend beyond the intermediate layer 5 laterally forming two longitudinal edge portions 8 at their respective longitudinal edges 3 and 4.

The longitudinal direction typically corresponds to the direction of formation of the continuous, web-like layered absorbent structure 14.

The two outer fibrous layers 1 and 2 are bonded together in the central region in which the intermediate layer 5 is present by the application of heat and moderate pressure to melt the particles 7 of thermoplastic, polymeric, organic material present in the intermediate layer 5, mixed with the particles 6 of hydrogelling, absorbent material, and 9 of odour control material.

The bond between the fibrous layers 1 and 2 is generated by the melting of the individual particles 7 of thermoplastic polymeric, organic material; as it melts, the polymeric material forms "bridges" connecting directly the fibrous layers 1 and 2 and/or also comprising particles 6 of absorbent gelling material, and particles 9 of odour control material.

It has to be noted that in the finished product the term "particulate material" identifies only the particles 6 of absorbent gelling material and 9 of odour control material, since the thermoplastic polymeric material after the melting step is no more in form of particles 7.

The overall surface area of the bond points represents a small fraction of the surface area of the fibrous layers 1 and 2 and of the particles of hydrogelling, absorbent material and odour control material, the characteristics of which thus remain almost unchanged.

Two continuous lines 10 of adhesive are also applied to the two sides of the intermediate layer 5 on the longitudinal edge regions 8 of the two outer fibrous layers 1 and 2 so as to prevent the particulate material, i.e. particles of hydrogelling, absorbent material 6 and of odour control material 9 from escaping from the longitudinal edges of the layered structure, which correspond to the superposed edges 3 and 4 of the two fibrous layers, and also to reinforce the connection between the fibrous layers themselves.

The containing layers and the particulate material can be alternatively bonded together by different means, other than the thermoplastic polymeric material in finely divided solid form, e.g. in form of particles 7 and the continuous lines of adhesive 10 described herein; for example, a sprayed layer of adhesive can replace at least the thermoplastic polymeric material in finely divided solid form.

The absorbent gelling material, which is preferably distributed in the form of particles 6, may be made of inorganic or organic substances such as cross-linked polymers, all known from the prior art. The odour control material can be any suitable odour control material known in the art, for example it can be constituted by particles of zeolite and silica.

The average dimensions of the particles 6 and 9, given as a weighted average of the smallest dimensions of the individual particles, can be between 50 microns and 1500 microns, preferably between 100 microns and 800 microns.

The quantity of the absorbent gelling material 6 in the intermediate layer 5 can range from 20 g/m², up to 600 g/m². The quantity of odour control material 9 can be between 40 g/m² and 200 g/m².

The thermoplastic, polymeric, organic material in finely divided solid form, e.g. in form of particles 7 has the purpose of bonding the two fibrous layers 1 and 2 together by melting and forming discrete, spaced-apart bond points between the fibres of the two layers. The thermoplastic, polymeric, organic material can also be used in other finely divided solid forms, e.g. in form of fibres.

As explained above, the bridges which form these bond points can involve particles of hydrogelling material and odour control material.

The quantity of thermoplastic, polymeric, organic material in finely divided solid form distributed and mixed with the hydrogelling, absorbent material can be between 5 g/m² and 180 g/m².

The thermoplastic, polymeric, organic material in finely divided solid form can preferably be melted at a temperature such as not to interfere with the characteristics of the other components of the layered structure, i.e. the fibrous layers and the particulate material, namely the hydrogelling, absorbent material and the odour control material. Therefore, the thermoplastic polymeric organic material must have fluidity characteristics such as to enable the necessary bonds to be formed rapidly.

These preferred characteristics can be achieved by a thermoplastic, polymeric, organic material in finely divided solid form having a melt flow index (M.F.I.), evaluated by the ASTM method D 1238-85 under conditions 190/2.16, of at least 25 g/10 min, preferably at least 40 g/10 min, and even more preferably at least 60 g/10 min.

If the layers 1 and 2 are made of a dry-formed short cellulose fibre material, it is preferable to use a thermoplastic, polymeric, organic material composed of particles of high-density polyethylene with maximum dimensions of about 400 microns, characterized by a melt flow index of about 50 g/10 min, of which the quantity distributed is between 12 g/m² and 90 g/m².

The continuous, web-like, layered absorbent structure 14 may also be formed by two different fibrous layers or may comprise more than two fibrous layers, and consequently more than one intermediate layer formed by the mixture of particulate material, e.g. particles of hydrogelling, absorbent material and of odour control material, and particles of thermoplastic, polymeric, organic material.

Of course, provided that a particulate material is actually comprised between containing layers, any of the absorbent gelling material, odour control material, or thermoplastic, polymeric, organic material in finely divided solid form comprised in a preferred layered structure made according to the present invention can be in a form which is different from the particulate form. For example, the absorbent gelling material can be in fibrous form, while the odour control material can be comprised as a solution sprayed onto a substrate; the thermoplastic, polymeric, organic material in finely divided solid form can also be in fibrous form, as already mentioned above.

The continuous lines 10 of adhesive disposed between the fibrous layers on the respective longitudinal edge portions prevent the particulate material forming the intermediate layer from escaping from the longitudinal edges of the structure. The structure therefore can be produced separately and stored as it is, for example, as a continuous strip rolled in the form of a roll 11, shown in Figure 2, which can subsequently be fed to the production line for disposable absorbent articles, for example sanitary napkins, where individual layered absorbent structures 12 are manufactured from the continuous, web-like, absorbent structure 14 in order to be incorporated as absorbent elements in the absorbent articles.

As illustrated in Figure 3, individual layered absorbent structures 12 of the desired length can be cut from a continuous, web-like, layered absorbent structure 14 of the type described above by means of cuts made along designated cutting lines 18 situated at predetermined intervals along the continuous structure 14 and indicated with a dashed line; as shown in Figure 3, the cuts are made in a direction transverse to the direction of feeding of the continuous structure 14 to the production line, which is indicated by an arrow and corresponds to the longitudinal direction of the continuous structure 14. In the embodiment illustrated in FIG. 3 the direction of the cut is perpendicular to the direction of feeding of the continuous structure 14, but non rectilinear cuts are also possible, for example curved cuts that provide each individual layered absorbent structure 12 with curved, usually convex, cut end edges. In the shown embodiment, cuts provide each individual layered absorbent structure 12 with an end surface 15 at each cut end edge. Individual layered absorbent structures 12, as well as the continuous web-like layered absorbent structure 14 from which they are achieved also have two major flat surfaces S substantially parallel to each other.

The individual layered absorbent structure 12 comprises a patch 40 of material, not illustrated in FIG. 3 for clarity, which is applied along each end surface 15 at each cut end edge. Each patch 40 provides joining between the containing layers 1 and 2 of the individual layered absorbent structure 12, and preferably provides the structure 12 with a seal at each end surface 15, therefore preventing the particulate material 6 and 9 from escaping from the cut edges of the individual layered absorbent structure 12. Loss or spillage of particulate material are therefore avoided.

FIGS. 4a to 4d show in detail different embodiments of the present invention, in which one or two patches of material 40 are applied to one of the end surfaces 15 of the individual layered absorbent structure 12 illustrated in FIG. 3. In all embodiments 4a to 4d the application of the patch 40 to the individual layered absorbent structure is achieved by means of an adhesive 42, e.g. a hot melt or a pressure sensitive adhesive, but any other means can also be used, for example heat sealing. The patch 40 has typically a rectangular form, having a length substantially corresponding to the width of the individual layered absorbent structure 12 along each cut end edge, and such a width as to join the two containing layers 1 and 2 at their end surface 15, and preferably partly extending onto at least a narrow portion of one or both of the major flat surfaces S of the individual layered absorbent structure 12 that are coterminous with the respective end surface 15, in order to achieve a better sealing action. It is preferable however that the area of the major flat surfaces S of the individual layered absorbent structures 12 which is interested by the application of the patch 40 of material is kept to a minimum in order to avoid possible interactions with the absorption characteristics and with the softness of the individual layered absorbent structures 12.

In FIGS. 4a and 4b only the containing layers 1 and 2 of the individual layered absorbent structure 12 are joined at an end surface 15 by means of a single patch 40, or of two patches 40, 40' of material, respectively, while in FIGS. 4c and 4d the containing layers 1 and 2 are both joined to an adjacent further layer 44 by a single patch 40 of material.

In FIG. 4a a single patch 40 of material is folded around the cut end edge of the individual layered absorbent structure 12 and adhered by means of a layer of adhesive 42 to the end surface 15 and to a narrow portion of both the major flat surfaces S of the individual layered absorbent structure 12 that are coterminous with the end surface 15.

FIG. 4b shows another embodiment that is similar to that of FIG. 4a, where the single patch 40 of material is substituted by two patches 40, 40', each one adhered at one end to a narrow portion of a respective major flat surface S of the individual layered absorbent structure 12 which is coterminous with the end surface 15, and to part of the end surface 15, and both adhered to each other at their other end, in order to provide said joining of the containing layers 1 and 2 along the end surface 15.

The embodiment of FIG. 4c is somewhat equivalent to that illustrated in FIG. 4a, but the patch 40 joins the containing layers 1 and 2 to an adjacent further layer 44 of the individual layered absorbent structure 12 that is coextensive with the containing layers 1 and 2 along the cut end edge defining the end surface 15. The further layer 44 could be an absorbent layer, such as a nonwoven or tissue layer, for example intended to acquire or diffuse fluid, or, alternatively, it could be a liquid impervious layer.

FIG. 4d shows still a further embodiment of the present invention in which the patch 40 of material joins the containing layers 1 and 2 to a further layer 44 that extends beyond the cut end edge forming the end surface 15.

Though all embodiments illustrated in FIGS. 4a to 4d refer to an individual layered absorbent structure 12 having rectilinear cut end edges, as that shown in FIG. 3, similar embodiments can also be achieved where curved cut end edges, and corresponding curved end surfaces 15 are provided on an individual layered absorbent structure 12.

The at least one patch 40 of material constitutes a layer that closes the respective end surface 15, therefore joining together the two fibrous layers 1 and 2 and preventing the particulate material 6 and 9 from escaping between them. Preferably the patch 40 effectively provided the respective end surface 15 with a seal.

It is not intended in the scope of the present invention that a patch 40 of material must totally entrap the particulate material at the respective end surface 15 of an individual layered structure 12, but rather that the patch 40 constitutes a joining means for the containing layers where the cut is made, entrapping a substantial percentage of the particulate material contained between them and therefore reducing the loss or spillage of particulate material. A minor loss of particulate material, e.g. through parts of the end surfaces where the patch of material is not applied, or through a patch constituted by a porous material, is within the scope of the present invention.

Any suitable material in sheet or layer form, e.g. those known for the manufacture of disposable absorbent articles, can be used for the patch 40 of material, such as for example nonwoven or tissue layers, either hydrophobic or hydrophilic, or alternatively polymeric films; such materials can be either fluid permeable or fluid impervious; preferably a nonwoven layer of synthetic fibres or a polymeric film can be used. The patch 40 can be made of a material comprising a single layer, or, alternatively, more than one layer.

In an example of the present invention an individual layered absorbent structure 12 of the type described previously and having a length of 207 mm is cut by rectilinear, transverse cuts from a continuous, web-like, layered absorbent structure wound in a roll as that illustrated in FIG. 2, having a width of 70 mm and an overall thickness of 1.5 mm; the individual layered absorbent structure 12 comprises:
- a first layer formed from a resin bonded air laid web of cellulose fibres 60 g/m²;
- an intermediate layer formed by a mixture of 63 g/m² of particles of absorbent gelling material, 61 g/m² of particles of zeolite, 87 g/m² of particles of silica, and 38 g/m² of particles of polyethylene;
- a second layer formed from a resin-bonded air laid web of cellulose fibres 60 g/m².

The two continuous lines of adhesive comprise two lines of hotmelt adhesive approximately 2 mm in width.

End surfaces are provided along the transverse cuts and correspond to the shorter sides of the individual layered absorbent structure 12.

A patch of a spunbonded nonwoven material made by 2.2 dtex, PE/PP bicomponent fibres and having a basis weight of 20 g/m², is provided along each end surface 15 of the individual layered absorbent structure 12 in a manner similar to that illustrated in FIG. 4a, said patch being adhered thereto by means of a hot melt adhesive. The patch has a length of about 70 mm corresponding to the entire width of the individual layered absorbent structure 12, while the width of each patch of nonwoven is about 20 mm, so that the patch extends about 10 mm in longitudinal direction on both major flat surfaces S of the individual layered absorbent structure 12 which are coterminous with the respective end surface 15, in order to join the two containing fibrous layers, and to provide a seal in said end surfaces 15.

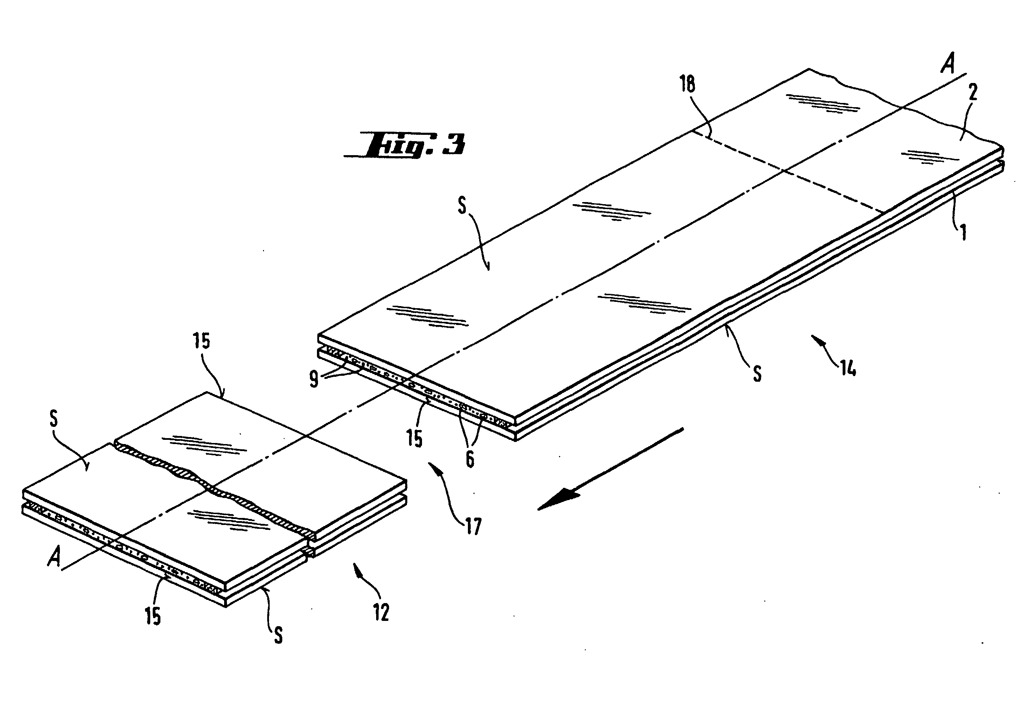

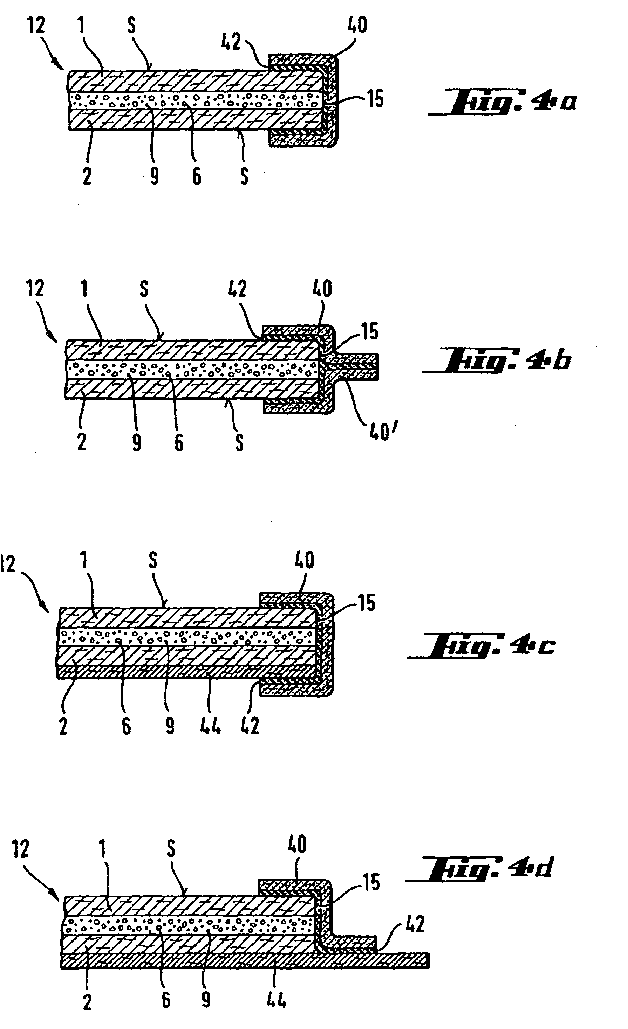

Figure 5 is a simplified diagram of a process for producing a disposable absorbent article, e.g. a sanitary napkin, that uses as absorbent element an individual layered absorbent structure 12 obtainable from a continuous, web-like, layered absorbent structure 14 fed as a semi-finished product.

The reel 20 supplies the continuous, web-like, layered absorbent structure 14 to the production line. The continuous structure 14 is cut into individual layered absorbent structures 12 at the cutting station 26, and the individual structures 12 are spaced out from each other in the direction of the production line in order to provide spaces 17 between the opposite end surfaces 15 of each pair of consecutive individual layered absorbent structures 12.

The amount of said spaces 17 can be varied according to the process conditions, as can be readily determined by the skilled man. In the illustrated embodiment the amount of the spaces 17 basically depends on the overall length of the finished disposable absorbent product, as compared to the length of each individual layered absorbent structure 12.

A fluid impervious backsheet 30 is then supplied to the production line from reel 34, and the individual layered absorbent structures 12 are associated to the backsheet 30 by known means, e.g. by adhesive means, not shown in the drawing for clarity.

A single larger patch 40 of material, supplied for example as a continuous band 48 of material from a reel 50, and then cut into patches 40 prior to application to the individual structures 12, is then applied at a station 46 along each pair of opposite end surfaces 15 belonging to consecutive individual structures 12. The patch is applied in a manner similar to that shown in FIG. 4d, wherein the backsheet 30 corresponds to the further layer adjacent to the containing layers 1 and 2, with the patch itself extending in longitudinal direction so as to completely cover a narrow portion of the major flat surfaces S of the two consecutive individual layered absorbent structures 12 that is coterminous with each respective end surface 15, and the exposed portion of the backsheet 30 comprised between the two layered structures 12, corresponding to the spaces 17. The application is achieved for example by means of an adhesive previously applied in any suitable known way to the patch itself or, alternatively, to the substrate, comprising the two end surfaces 15, the narrow portions of the major flat surfaces S, and the exposed portion of the backsheet 30. The application of the patch 40 involves the backsheet 30 associated to the individual layered absorbent structures 12 in correspondence of each space 17 comprised between two consecutive individual layered absorbent structures 12. Therefore the patch 40 performs the further action of joining the individual layered absorbent structures 12 to the associated backsheet 30 at both end edges also, which correspond to the end surfaces 15.

After the application of the patches 40 the backsheet 30 with the individual layered absorbent structures 12 associated and joined thereon are fed to an assembly station where a fluid pervious topsheet 28 is supplied from reel 32. The topsheet 28 and the backsheet 30 incorporate the individual structures 12 therebetween, and are finally joined together and cut along a perimeter at a cutting and sealing station 38 to form sanitary napkins 36 each comprising an individual layered absorbent structure 12 sealed at both end surfaces 15 corresponding to the transverse cuts. The material of the patches 40 is coterminous with the topsheet 28 and the backsheet 30 at the longitudinal ends of the absorbent article, and therefore it is preferably constituted by a polymeric film or by a hydrophobic nonwoven material in order to avoid possible transmission of fluid from the end surfaces 15 of the individual layered absorbent structure 12 towards the longitudinal ends of the absorbent article 36.

According to an alternate embodiment of the process of the present invention a separate shorter patch 40 of material can be applied to each end surface 15 of the individual layered absorbent structure 12 instead of the longer patch covering two opposite end surfaces 15 belonging to consecutive structures 12, therefore achieving an embodiment exactly corresponding to that illustrated in FIG. 4d.

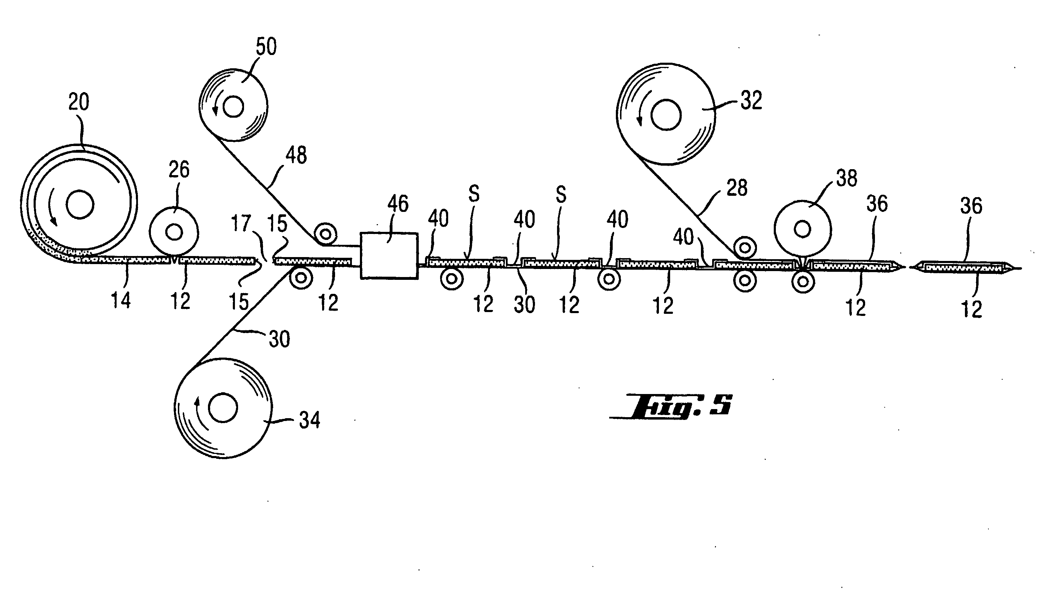

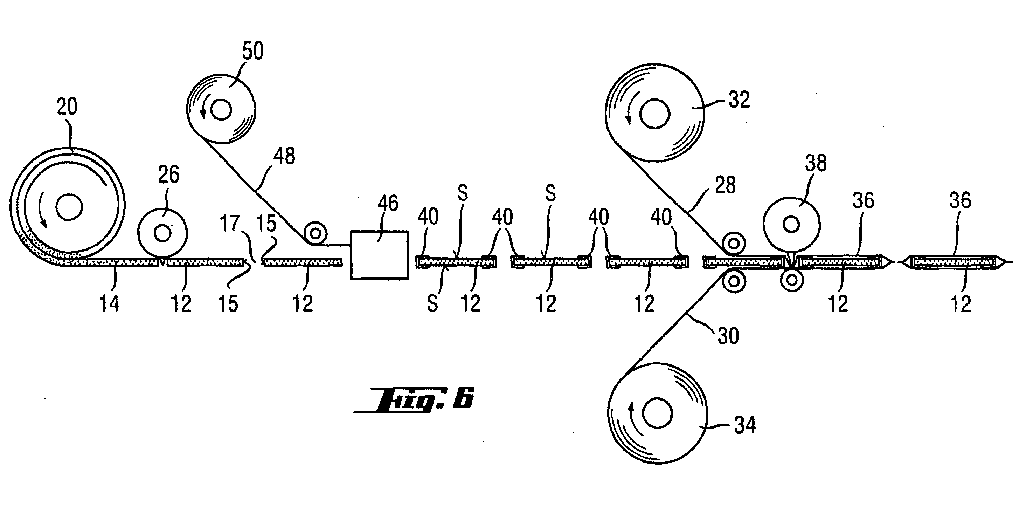

A further alternative embodiment of the process of the present invention is illustrated in FIG. 6, where a patch 40 of material is applied to each end surface 15 of the individual layered absorbent structure 12 in an arrangement equivalent to that illustrated in FIG. 4a, before the individual layered structures 12 are associated to the backsheet 30. In this embodiment both the backsheet 30 and the topsheet 28 are associated to the individual layered absorbent structures 12 at the assembly station.

Although the containing layers of the layered structures described so far all have the same width which is constant along the length of the structure, further embodiments are also possible in which the containing layers have different widths, or, also, in which the width of the layered structure can vary along the length of the structure itself, in order to provide, for example, a shaped absorbent element for a disposable absorbent article, e.g. an hourglass shaped one, which can be manufactured from a continuous layered structure having a width varying along the longitudinal direction.

While the present invention has been described in association with layered structures comprising two containing layers and a particulate material comprised therebetween, it can be also applicable to different layered structures, e.g. to structures having a different number of superimposed layers, or, alternatively, to another known type of C-folded absorbent laminate consisting of a single fibrous layer folded twice on itself and sealed by means of adhesive along the overlapping longitudinal side margins; the particulate material, e.g. absorbent gelling material and odour control material, is comprised therebetween. The particulate material and the superimposed portions of the fibrous layer are bonded together by means of sprayed adhesive. This type of structure is typically made on the production line of a sanitary napkin, rather than being produced as a semi-finished product, i.e. as a continuous, web-like layered structure to be subsequently fed to the production line. It does not have loss or spillage of particulate material along the longitudinal edges, but still has this problem at its end surfaces where it is actually cut in transverse direction in the production line in order to form the individual layered structures that are to be incorporated in the sanitary products.

In an alternative embodiment of the process of the present invention the process can also be used to produce individual layered structures by cutting a larger continuous layered structure along both longitudinal and transverse cutting lines and therefore providing the individual layered structures with end surfaces between the longitudinal and transverse cuts. Patches of material can be subsequently applied to all end surfaces of each individual layered structure, i.e. along the entire cut, therefore providing each individual layered structure with joining between the containing layers both in transverse and in longitudinal directions.

## Claims

1. An individual layered structure (12) for use in a disposable absorbent article comprising a particulate material (5) contained between containing layers (1, 2), said containing layers (1, 2) in the form of two continuous strips of the same width, which are superposed so that their respective longitudinal edges (3, 4) coincide, at least one of said layers (1, 2) being permeable to liquids, said individual layered structure (12) being cut from a larger structure (14) and having said containing layers (1, 2) joined along at least part of said cut, and further having end surfaces (15) along said cuts, said individual layered structure (12) being **characterized in that** it comprises at least one patch (40) of material applied along and to at least part of said end surfaces (15), said at least one patch (40) of material providing joining between said containing layers (1, 2) in said at least part of said end surfaces (15) where it is applied.

2. An individual layered structure (12) according to claim 1, **characterized in that** said at least one patch (40) of material is a polymeric film.

3. An individual layered structure (12) according to any preceding claim, **characterized in that** said at least one patch (40) of material is a nonwoven layer.

4. An individual layered structure (12) according to any preceding claim, **characterized in that** it comprises a further layer (44) adjacent to said containing layers (1, 2), wherein said at least one patch (40) of material joins said containing layers (1, 2) to said adjacent further layer (44).

5. An individual layered structure (12) according to claim 4, **characterized in that** said adjacent further layer (44) extends beyond said end surfaces (15) of said containing layers (1,2) along at least the part of said end surfaces (15).

6. An individual layered structure (12) according to any preceding claim, **characterized in that** said joining provided by said at least one patch (40) of material between said containing layers (1, 2) constitutes a seal.

7. An individual layered structure (12) according to any preceding claim, **characterized in that** said containing layers (1, 2) are fibrous layers, preferably absorbent fibrous layers.

8. An individual layered structure (12) according to any preceding claims, **characterized in that** said particulate material comprises a thermoplastic polymeric material (7) in finely divided form.

9. A process for manufacturing individual layered structures (12) according to claims 1 to 3, comprising the steps of:
a) providing a continuous web-like layered structure (14) constituting said larger structure from which said individual layered structures (12) are cut, said continuous, web-like layered structure (14) comprising a particulate material between containing layers (1,2), said containing layers (1, 2) in the form of two continuous strips of the same width, which are superposed so that their respective longitudinal edges (3, 4) coincide, at least one of said layers (1, 2) being permeable to liquids;
b) cutting said continuous web-like layered structure (14) to provide said individual layered structures (12) having end surfaces (15) along said cuts;
said process being **characterized in that** it further comprises the step of:
c) applying at least one patch (40) of material along and to at least part of said end surfaces (15), so that said at least one patch (40) of material provides joining between said containing layers (1, 2) in said at least part of said end surfaces (15).

## Patentansprüche

1. Einzelne, geschichtete Anordnung (12) zur Verwendung in einem wegwerfbaren, absorbierenden Artikel mit einem partikelförmigen Material (5), das zwischen einschließenden Lagen (1, 2) eingeschlossen ist, wobei die einschließenden Lagen (1, 2) in der Form von zwei kontinuierlichen Streifen der gleichen Breite vorliegen, welche derart aufeinanderliegen, dass ihre jeweiligen Längskanten (3, 4) zusammenfallen, wobei mindestens eine der Lagen (1, 2) Flüssigkeits-durchlässig ist, wobei die einzelne, geschichtete Anordnung (12) aus einer größeren Anordnung (14) geschnitten ist und die einschließenden Lagen (1, 2) in entlang mindestens eines Abschnitts des Schnitts verbundenem Zustand aufweist und die ferner End-Oberflächen (15) entlang der Schnitte aufweist, wobei die einzelne, geschichtete Anordnung (12) **dadurch gekennzeichnet ist, dass** diese mindestens ein Material-Stück (40) aufweist, das entlang und auf mindestens einem Abschnitt der End-Oberflächen (15) aufgebracht ist, wobei das mindestens eine Material-Stück (40) eine Verbindung zwischen den einschließenden Lagen (1, 2) in mindestens dem Abschnitt der End-Oberflächen (15) schafft, wo dieses aufgebracht ist.

2. Einzelne, geschichtete Anordnung (12) gemäß Anspruch 1, **dadurch gekennzeichnet, dass** das mindestens eine Material-Stück (40) eine polymere Folie ist.

3. Einzelne, geschichtete Anordnung (12) gemäß einem der vorherigen Ansprüche, **dadurch gekennzeichnet, dass** das mindestens eine Material-Stück (40) eine Vlieslage ist.

4. Einzelne, geschichtete Struktur (12) gemäß einem der vorherigen Ansprüche, **dadurch gekennzeichnet, dass** diese eine weitere Lage (44) aufweist, die benachbart zu den einschließenden Lagen (1, 2) angeordnet ist, wobei das mindestens eine Material-Stück (40) die einschließenden Lagen (1, 2) mit der benachbart angeordneten, weiteren Lage (44) verbindet.

5. Einzelne, geschichtete Struktur (12) gemäß Anspruch 4, **dadurch gekennzeichnet, dass** die benachbart angeordnete, weitere Lage (44) jenseits der End-Oberflächen (15) der einschließenden Lagen (1, 2) entlang mindestens dem Abschnitt der End-Oberflächen (15) verläuft.

6. Einzelne, geschichtete Anordnung (12) gemäß einem der vorherigen Ansprüche, **dadurch gekennzeichnet, dass** die Verbindung, die durch das mindestens eine Material-Stück (40) zwischen den einschließenden Lagen (1, 2) geschaffen ist, einen Verschluss bildet.

7. Einzelne, geschichtete Struktur (12) gemäß einem der vorherigen Ansprüche, **dadurch gekennzeichnet, dass** die einschließenden Lagen (1, 2) Faserlagen, vorzugsweise absorbierende Faserlagen sind.

8. Einzelne, geschichtete Anordnung (12) gemäß einem der vorherigen Ansprüche, **dadurch gekennzeichnet, dass** das partikelförmige Material ein thermoplastisches, polymeres Material (7) in fein geteilter Form aufweist.

9. Verfahren zur Herstellung einzelner, geschichteter Anordnungen (12) gemäß den Ansprüchen 1 bis 3, umfassend die Schritte:
a) Bereitstellen einer kontinuierlichen, gewebeartigen, geschichteten Anordnung (14), die die größere Anordnung bildet, aus welcher die einzelnen, geschichteten Anordnungen (12) geschnitten werden, wobei die kontinuierliche, gewebeartige, geschichtete Anordnung (14) ein partikelfömriges Material zwischen einschließenden Lagen (1, 2) aufweist, wobei die einschließenden Lagen (1, 2) in der Form von zwei kontinuierlichen Streifen der gleichen Breite vorliegen, die derart aufeinanderliegen, dass ihre jeweiligen Längskanten (3, 4) zusammenfallen, wobei mindestens eine der Lagen (1, 2) Flüssigkeits-durchlässig ist;
b) Schneiden der kontinuierlichen, gewebeartigen, geschichteten Anordnung (14), um die einzelnen, geschichteten Anordnungen (12) mit End-Oberflächen (15) entlang der Schnitte bereitzustellen;
wobei das Verfahren **dadurch gekennzeichnet ist, dass** dieses ferner den Schritt umfasst:
c) Aufbringen mindestens eines Material-Stücks (40) entlang und auf mindestens einen Abschnitt der End-Oberflächen (15), so dass das mindestens eine Material-Stück (40) eine Verbindung zwischen den einschließenden Lagen (1, 2) in mindestens dem Abschnitt der End-Oberflächen (15) schafft.

## Revendications

1. Structure individuelle à couches (12) utilisable dans un article absorbant jetable comprenant un matériau particulaire (5) retenu entre des couches de retenue (1, 2), lesdites couches de retenue (1, 2) se présentant sous la forme de deux bandes continues de même largeur qui sont superposées de manière que leurs bords longitudinaux (3, 4) respectifs coïncident, au moins une desdites couches (1, 2) étant perméable aux fluides, ladite structure individuelle à couches (12) étant découpée à partir d'une structure plus grande (14), lesdites couches de retenue (1, 2) sont réunies au moins le long d'une partie de ladite découpe, et présentant, en outre, des surfaces d'extrémité (15) le long desdites découpes, ladite structure individuelle à couches (12) étant **caractérisée en ce qu'**elle comprend au moins une pièce (40) de matériau appliquée le long et au moins à une partie desdites surfaces d'extrémité (15), au moins ladite pièce (40) de matériau réalisant une liaison entre lesdites couches de retenue (1,2) au moins dans ladite partie desdites surfaces d'extrémité (15) où elle est appliquée.

2. Structure individuelle à couches (12) selon la revendication 1, **caractérisée en ce qu'**au moins ladite pièce (40) de matériau est un film polymère.

3. Structure individuelle à couches (12) selon l'une quelconque des revendications précédentes, **caractérisée en ce qu'**au moins ladite pièce (40) de matériau est une couche non tissée.

4. Structure individuelle à couches (12) selon l'une quelconque des revendications précédentes, **caractérisée en ce qu'**elle comprend une autre couche (44) adjacente auxdites couches de retenue (1, 2), dans laquelle au moins ladite pièce (40) de matériau réunit lesdites couches de retenue (1, 2) à ladite autre couche (44) adjacente.

5. Structure individuelle à couches (12) selon la revendication 4, **caractérisée en ce que** ladite autre couche (44) adjacente s'étend au-delà desdites surfaces d'extrémité (15) desdites couches de retenue (1, 2) au moins le long de la partie desdites surfaces d'extrémité (15).

6. Structure individuelle à couches (12) selon l'une quelconque des revendications précédentes, **caractérisée** en ce ladite liaison réalisée au moins par ladite pièce (40) de matériau, entre lesdites couches de retenue (1, 2), constitue un joint d'étanchéité.

7. Structure individuelle à couches (12) selon l'une quelconque des revendications précédentes, **caractérisée** en ce lesdites couches de retenue (1, 2) sont des couches fibreuses, de préférence, des couches fibreuses absorbantes.

8. Structure individuelle à couches (12) selon l'une quelconque des revendications précédentes, **caractérisée en ce que** ledit matériau particulaire comprend un matériau polymère thermoplastique (7) sous une forme finement divisée.

9. Procédé pour fabriquer des structures individuelles à couches (12) selon les revendications 1 à 3, comprenant les étapes consistant à :
(a) fournir une structure à couche de type nappe (14), continue, constituant ladite structure plus grande à partir de laquelle lesdites structures individuelles à couches (12) sont découpées, ladite structure à couches de type nappe (14), continue comprenant un matériau particulaire entre lesdites couches de retenue (1, 2), lesdites couches de retenue (1, 2) se présentant sous la forme de deux bandes continues de même largeur qui sont superposées de manière que leurs bords longitudinaux (3, 4) respectifs coïncident, au moins une desdites couches (1, 2) étant perméable aux liquides ;
(b) découper ladite structure à couches de type nappe (14), continue, afin de fournir lesdites structures individuelles à couches (12) présentant des surfaces d'extrémité (15) le long desdites découpes ;
ledit procédé étant **caractérisé en ce qu'**il comprend, en outre, l'étape consistant à :
(c) appliquer au moins une pièce (40) de matériau le long et au moins à une partie desdites surfaces d'extrémité (15), afin qu'au moins ladite pièce (40) de matériau réalise une liaison entre lesdites couches de retenue (1, 2) au moins dans ladite partie desdites surfaces d'extrémité (15).
